# EUROPEAN PATENT APPLICATION

(11) **EP 2 072 511 A1**
(43) Date of publication of application: **24.06.2009**
(21) Application number: 07124044.4
(22) Date of filing: 21.12.2007
(51) Int. Cl.: C07D 409/04

(54) **Method useful for amplifying, detecting and depleting pathologic forms of the cellular prion protein**

(71) Applicant: INSERM (Institut National de la Santé et de la Recherche Médicale), 75654 Paris Cedex 13 (FR)
(72) Inventor: The designation of the inventor has not yet been filed
(74) Representative: Le Coupanec, Pascale A.M.P.

(57) **Abstract**

The present invention relates to a method for selectively amplifying the oligomerization of at least one PrP^{Sc} isoform of the PrP^{C} cellular prion protein, characterized in that it comprises bringing said PrP^{Sc} isoform, under conditions suitable for amplification, together with an effective amount of at least one compound of general formula I as follow:

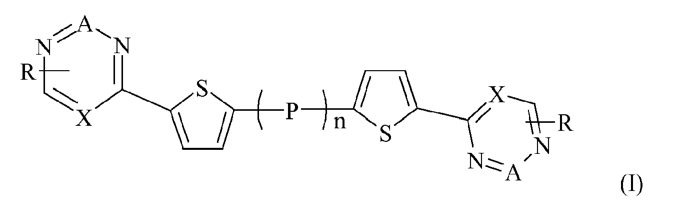

The instant invention is also directed to new compounds of formula I and pharmaceutical compositions including them.

## Description

The present invention relates principally to the use of some derivatives for the diagnosis, characterization, clinical monitoring, etc., of encephalopathy, in particular of transmissible subacute spongiform encephalopathies (TSSEs) including bovine spongiform encephalopathies (BSEs). It also relates to some of these derivatives as new compounds particularly interesting for their ability to efficiently and selectively stimulate the oligomerization of the infectious titer of PrP^{Sc} isoforms of the cellular prion protein PrP^{C}.

Prion diseases or transmissible subacute spongiform encephalopathies (TSSEs) are fatal neurodegenerative conditions which affect both humans and animals. TSSEs include essentially Creutzfeldt-Jakob disease, in humans (CJD), scrapie in sheep and goat and bovine spongiform encephalopathy (BSE) in cattle. Other encephalopathies have been demonstrated in felines, in mink or in certain wild animals, such as deer or moose.

The infectious agent responsible for these diseases is unconventional. Currently, the dominating hypothesis is that of "prions", according to which the cellular prion protein, PrP^{C}, synthesized by the host organism, would be capable of adopting a pathological conformation, called PrP^{Sc}, which denotes the "scrapie" isoform.

These two isoforms have the same amino acid sequence but differ in terms of their secondary structure: PrP^{Sc} has a significantly higher content of beta-pleated sheets, whereas normal PrP (PrP^{C}) has a greater number of alpha helices.

The accumulation of PrP^{Sc} in the brain of individuals or animals suffering from TSSE causes degeneration of said brain. Research on these diseases has shown a remarkable increase in development since the mad cow crisis in Europe and the appearance of the new variant of Creutzfeldt-Jacob disease in humans (vCJD). Progress has, as a result, been made in terms of the knowledge of these unconventional infectious agents. However, at the current time, no available treatment exists for treating patients suffering from CJD, nor is there a test for early diagnosis.

In diagnostic terms, the most recent methods are based on a selected detection of abnormal PrP (PrP^{Sc}), associated with the infectious agent, by taking advantage of its partial protease resistance. In this respect, it is possible to distinguish Western blotting methods, which are based on the immunodetection of PrP^{Sc} in a tissue extract, (Schaller O. et al., Acta Neuropathol., 1999, 98, 437-443), and ELISA assays, which also involve treatment of the tissue extracts with a protease.

The document WO 01/35104 has proposed a test involving, successively, the treatment of a sample presumed to contain PrP^{Sc} with proteinase K under conditions adjusted so as to allow complete degradation of the PrP^{C} protein while at the same time conserving all or part of the repeated octapeptide motifs of the PrP^{Sc} protein, the subsequent bringing of said sample thus treated into contact with a ligand, in particular an antibody specific for the octapeptide motifs, and the possible detection of a repeated octapeptide motifs-ligand complex. WO 02/04954 describes a method for detecting prion using a step for amplifying the PrP^{Sc} protein by bringing abnormal PrP into contact with a known amount of normal PrP.

Among the actually available methods, mention may be made of the kit Prionics from the Prionics firm based on an immunological detection of the PrP^{Sc} and the technic PCMA (protein Misfolding Cyclic Amplification) developed by Amprion Inc firm which consist to reproduce the replication cycle of prions in assay tube.

However, some tests have the drawback of lacking sensitivity and, consequently, producing false negatives. Furthermore, they are carried out post-mortem. In addition, false positives samples could be detected according to the recent results from Deleault et al. (2007, PNAS, 104(23): 9741) which demonstrated using the PMCA technique that they can amplify PrP^{Sc} using polyARNA and normal brain homogenates.

More recently, the inventors disclosed in the document EP 1 731 911, a method of detection which, in opposite to the previous ones, which could be applied in living beings, which are rapid and which make it possible to detect the pathology at a early stage. More particularly, the method is based on the use of specific compounds which allow an efficient detection of the PrP^{Sc} form because their ability to selectively stimulate the oligomerisation of this form.

Unexpectedly, the inventors have now discovered that it is possible to improve significantly such diagnosis method with the proviso of using new derivatives.

An object of the present invention is precisely to propose new derivatives which would make possible to establish an early diagnosis of the disease, i.e. ante-mortem, in humans or animals, and which is non invasive.

According to one of its aspects, the present invention is principally directed to a method for selectively amplifying the oligomerization of at least one PrP^{Sc} isoform of the PrP^{C} cellular prion protein, characterized in that it comprises bringing said PrP^{Sc} isoform, under conditions suitable for amplification, together with an effective amount of at least one compound of general formula I as follow: 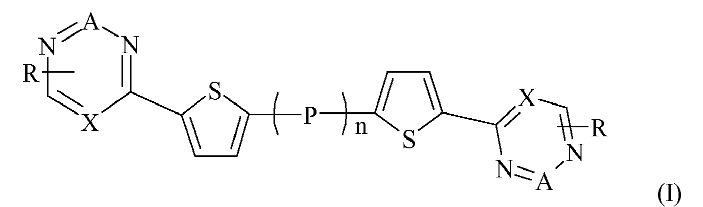 in which:
- A, independently of one another, represent a nitrogen, a -CR₁-, -CO(R₁) -,
- CS(R₁)-, -CN(R₁)(R₂)- group with R₁ and R₂ representing, independently of one another, a hydrogen atom, a saturated or unsaturated, linear, branched or cyclic, C₁ to C₁₂ alkyl radical, where appropriate substituted,
- X, independently of one another, represent a nitrogen atom or CR₁" with R₁" representing a group as defined above for R₁ and in particular an hydrogen,
- R independently of one another, represent a hydrogen atom or a radical chosen from:
   - a OR'₁, N(R'₁)(R'₂), SR'₁ group with R'₁ and R'₂ representing, independently of one another, a group as defined above for R₁ and/or R₂,
   - a saturated or unsaturated, linear, branched or cyclic, C₁ to C₁₂ hydrocarbon-based radical, the hydrocarbon-based chain of which may, where appropriate, be interrupted with one or more heteroatoms chosen from S, O and N atoms, and where appropriate, substituted for example by a OR'₁, N(R'₁)(R'₂), SR'₁ group as defined previously,
- P represents an aromatic nucleus C₅ to C₁₆ or a saturated or unsaturated linear, branched or cyclic C₁ to C₁₂ alkyl divalent group, the hydrocarbon-based chain of which may, where appropriate, be interrupted with one or more heteroatoms chosen from S, O, N and Si atoms, and/or with one or more cyclic alkyl ring, aryl ring or heteroaryl ring and where appropriate, substituted,
- n is zero or an integer equal or greater to 1, in particular is different from zero and no greater than 6,
- and salts, solvates, isomers or derivatives thereof.

In the context of the present invention, the terms "aromatic nucleus", means an aryl or an heteroaryl ring.

The term "aryl" denotes a 5- or 6-membered aromatic ring or a bi-, tri- or tetra-cyclic aromatic ring. For example, the aryl group may be chosen from the phenyl, naphthyl, phenanthryl, fluorenyl, pyrenyl and anthryl group.

The term "heteroaryl" denotes a 5- or 6-membered aromatic ring comprising 1 or 2 heteroatoms or a bi- or tri-cyclic aromatic nucleus comprising from 1 to 4 heteroatoms, in particular with the fused rings having 5 or 6 ring members. For example, the heteroaryl group may be chosen from pyrrolyl, imidazolyl, pyridyl, pyrimidinyl, carbazolyl, pyrazinyl, indolyl, indazolyl, furyl, pyranyl, thiazolyl, thiopuranyl and thienyl ring.

In the context of the present invention, the terms "aromatic nucleus", "aryl", and "heteroaryl" include all the positional isomers.

As stated previously, said aromatic nucleus may be substituted. According to a specific embodiment, they can include at least one saturated or unsaturated, linear, branched or cyclic, C₁ to C₁₂ hydrocarbon-based radical, the hydrocarbon-based chain of which may, where appropriate, be interrupted with one or more heteroatoms chosen from S, O and N atoms, and where appropriate, substituted, in particular in end terminal, for example by a OR'₁, N(R'₁)(R'₂), SR'₁ group and more particularly by N(R'₁)(R'₂), as defined previously.

Unexpectedly, and as proven with the results submitted here-after, the inventors have observed that the compounds of general formula I are particularly effective for selectively amplifying the oligomerization of the PrP^{Sc} isoforms of the cellular prion protein, in particular in the presence of the nonpathogenic form thereof. Thus, the compounds of general formula I have the particularity of increasing the presence of oligomeric forms of PrP^{Sc} without increasing those of the normal PrP^{C} isoform. Quite obviously, the compounds in accordance with the invention act specifically on the PrP^{Sc} molecules.

This ability is particularly advantageous insofar as it would make it possible to diagnose at an early stage the risk of the occurrence of an encephalopathy.

By selectively amplifying, in a biological sample, PrP^{Sc} isoforms that are not detectable, as such, with conventional diagnosis methods, detection at a significantly earlier stage could be possible.

For obvious reasons, such an early diagnosis can only be beneficial in curative and economic terms, and constitute a major need in terms of public health and with respect to sanitary safety.

Thus, the invention makes it possible to develop a test for detecting prions in a physiological fluid, such as urine and blood, for the preclinical diagnosis of scrapie in sheep and of bovine spongiform encephalopathy, and thus to detect sick animals which can currently pass into the food chain.

Furthermore, these compounds have the other advantage to intrinsincly possess fluorescent properties. For the reasons submitted here-after, such properties are particularly advantageous for the diagnosis.

According to another of its aspects, the present invention is directed toward a method useful for detecting a PrP^{Sc} isoform of the PrP^{C} cellular prion protein, comprising at least the implementation of a method of the invention and a step of detection of said isoform.

The detection step may be direct by taking advantage of the native fluorescent of the compounds of general formula (I) and/or can be carried out by a method of hybridization with nucleotides or antibody/antigen interaction type.

According to another of its aspects, the present invention relates to a method useful for detecting the presence or the risk of the occurrence of an encephalopathy in an animal or human individual, said method comprising at least:
- bringing a biological sample from said individual together with an effective amount of at least one compound of general formula (I) as defined above, under conditions suitable for the amplification of the oligomerization of PrP^{Sc} isoform(s) possibly present in said sample, and
- detecting the possible presence of said PrP^{Sc} isoform, indicative of the presence or of the risk of development of an encephalopathy in said individual.

The method according to the invention is particularly useful for detecting a transmissible subacute spongiform encephalopathy (TSSE), and especially a Creutzfeldt-Jakob disease in humans, scrapie in sheep and goat, and bovine spongiform encephalopathy.

In addition, the methods according to the invention applied to blood samples give a better guarantee with regard to human blood transfusions and make it possible to establish a test for the early diagnosis of TSSEs in humans, or even the optimization of future treatments.

According to a specific embodiment, the compound of general formula I can be used in a form associated with a marker suitable for luminescent detection or detection by affinity.

According to another specific embodiment of the invention, the detection step involves an immunodetection process, in particular using specific antibodies.

According to this second variant, this detection comprises at least the treatment of all or part of said sample with a protease, such as, for example, protease K, so as to destroy the normal PrP isoform, the separation of the proteins and the detection of the PrP^{Sc} isoform possibly present, in particular using a specific antibody. The step of destruction of the normal PrP isoform is generally carried out after the step of the PrP^{Sc} isoform oligomerization amplification.

A mixture of antibodies SAF60 and SAF69, specific for the sequences 157-161 of human PrP, and SAF70, specific for the sequence 156-162 of human PrP, is in particular suitable for the detection.

Similarly, the treatment with proteinase K can be carried out under the conditions described in document WO 01/35104, which result in complete degradation of the PrP^{C} protein while at the same time conserving all or part of the repeated octapeptide motifs of the PrP^{Sc} protein. In this case, the detection can be carried out by subsequently bringing said sample thus treated into contact with a ligand, in particular octapeptide motifs, for detecting the possible presence of repeated octapeptide motifs-ligand complex(es).

According to yet another of its aspects, the present invention relates to a kit for selectively amplifying the oligomerization of at least one PrP^{Sc} isoform and also to a kit useful for diagnosing TSSEs, comprising, as a reagent, at least one compound of general formula I.

According to a specific embodiment, a compound of general formula (I) may be furthermore used for the depletion in biological liquid of the pathogen PrP^{Sc} isoform. Such a application is based on the use of solid support like for example resin, beads or filter on the surface of which at least one compound of formula I has been fixed. The so-obtained support is contacted with a liquid medium like for example the blood, a serum or urine, in conditions suitable for promoting the trapping of pathogen form, if present. Such a method is related to the filtration or capture of prions.

### Compound of general formula I

As specified above, the invention is based on the use of the compound of general formula I as follows:

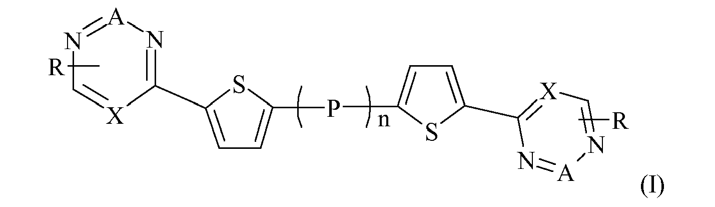

According to one embodiment, P is an insaturated linear or branched C₂ to C₁₂ alkyl divalent group, optionally interrupted by an heteroatom, a C₃ to C₁₂ cyclic alkyl and/or an aromatic nucleus as defined previously.

In particular, said alkyl radical represent a C₂ to C₈ alkenyl group optionally interrupted by an heteroatom and /or a cyclic, aryl or heteroaryl group as defined previously like for example a thienyl or carbazolyl ring.

In particular, n is in this specific case more particularly equal to 1.

According to another embodiment, P is an aromatic nucleus as defined previously.

In particular, P is a 5- or 6-membered aromatic ring comprising 1 or 2 heteroatoms or a bi- or tri-cyclic aromatic ring comprising from 1 to 4 heteroatoms.

For example, P may be chosen from pyrroryl, imidazolyl, pyridyl, pyrimidinyl, carbazolyl, pyrazinyl, indolyl, indazolyl, furyl, pyranyl, thiazolyl, thiopuranyl and thienyl ring.

More particularly, P is an heteroaromatic nucleus as defined previously and in particular a carbazolyl ring or an heteroaryl ring including at least one sulphur atom.

Preferably, the heteroaryl does not comprise an oxygen atom. More preferably, the heteroaryl comprises only sulphur as heteroatom(s).

In particular, P represents a 5 or 6-membered aromatic ring comprising from 1 to 2 sulphur atom. More particularly, P represents a thienyl ring.

According to one embodiment, radicals R are in the meta- or para-position with respect to the groups A. In particular, when a radical R is present on a nitrogen atom of the pyrimidyl ring, it is preferably a hydrogen atom.

As regards A, they are more particularly a -CN(R₁)(R₂)- with R₁ and R₂ representing an alkyl radical as defined above or an hydrogen.

As regards R, they are more particularly a N(R'₁)(R'₂) group with R'₁ and R'₂ representing, independently of one another, a group as defined above for R₁ and/or R₂, or a saturated or unsaturated, linear, branched or cyclic, C₁ to C₁₂ hydrocarbon-based radical, the hydrocarbon-based chain of which may, where appropriate, be interrupted with one or more heteroatoms chosen from S, O and N atoms, and substituted in end terminal by a N(R'₁)(R'₂) group as defined previously.

As regards the salts thereof, they may be any of the inorganic or organic salts insofar as they are found to be compatible with the implementation of the invention.

As regards the derivatives thereof, they may more particularly be, for the purpose of the invention, compounds of general formula (I) incorporating into their structure at least one entity, which may or may not belong to a labeling system, capable of providing them with a method of detection, for example luminescent detection, like for example a signal that is visible in the infrared range or by fluorescence, for instance Europium, or detection by affinity, such as biotin labeling in the case of streptavidin/biotin recognition.

The entity, which is generally chemical, intended to provide the compound of general formula I with a method of labeling for detection, can be attached to the molecule of general formula I by conventional methods.

Of course, its location is selected so as not to affect the expected reactivity of the derivative of general formula I thus obtained.

The derivatives thereof may be also compounds of general formula (I) including into their structure at least one entity capable of promoting a linkage to a solid support. Such a embodiment may be in particular illustrated by compounds of formula I including as P spacer a carbazole group where the central nitrogen atom is functionalized by an amino alkyl group in order to react with an activated polysaccharide surface such as dextran. According to a variant of the invention, the compound of general formula I are of formula I' as follow: 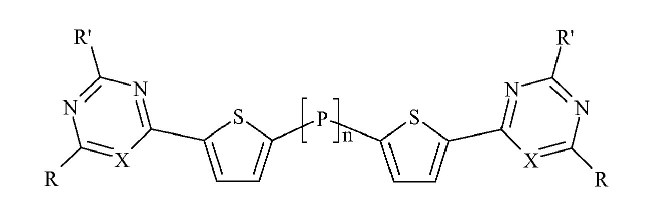 with R, P, n and X being as defined in formula I and R' being a radical chosen among NR'₁R'₂ and SR'₁ with R'₁ and R'₂ being as defined previously.

More particularly, R represent a N(R'₁)(R'₂) with R'₁ and R'₂ being as defined previously.

More particularly, X is a nitrogen.

As regards P, it is more particularly a thienyl where appropriate substituted.

According to a specific embodiment, n is equal at least to 2.

According to a preferred embodiment, at least one of the radicals R and R' is different from hydrogen.

In another preferred embodiment, R and R' have the same meaning.

In another preferred embodiment, R and R are identical and represent an amino, in particular an -NH₂ radical.

Are particularly interesting in the methods according to the invention the compounds of formula (I) or (I') including at least 2 thienyl ring i.e wherein P is a thienyl ring and n is equal to 2.

The compouds of formula (I) and (I') may be substituted.

As possible substituents, mention may more particularly be made of halogen atoms especially chosen from fluorine, bromine, iodine and chlorine atoms, and more particularly bromine; OH, OR', NR'R", SR' and R' radical with R' and R" representing, independently of one another, a linear or branched C₁ to C₁₀ alkyl radical; saturated, unsaturated or aromatic C₄ to C₈, in particular C₅ to C₆, heterocyclic radicals, optionally substituted, comprising one or more heteroatoms chosen from N, O and S, for example a thienyl radical, a carbazoyl radical or a thiazolyl radical.

According to a specific embodiment, a substituent may advantageously be chosen from a bromine or a thienyl radical.

By way of nonlimiting representation of the compounds of general formula I, mention may more particularly be made of 6,6'-(2,2':5',2":5",2'''-quaterthiophene-5,5'''-diyl)bis(1,3,5-triazine-2,4-diamine), 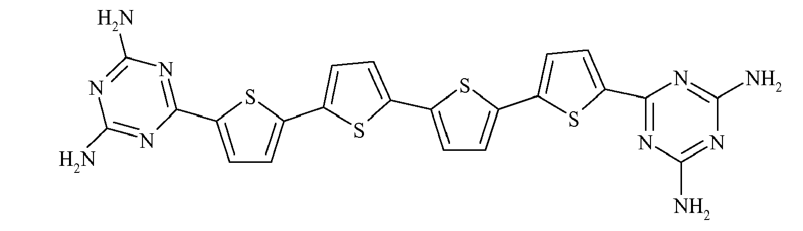 and salts or derivatives thereof.

Given their reactivity with respect to the PrP^{Sc} isoforms, the compounds of general formula I manifestly have the ability to bind to at least one amino acid.

Without wishing to be bound to any theory, it would appear that the compounds in accordance with the present invention are found to be capable of binding to at least one of the following two regions of the C-terminal part of the PrP protein:
- a region located at the disulfide bridge (residues Cys 179 and Cys 214) which connects the H2 and H3 helices, and
- a region located in the H1 helix (comprising residues 144-154), located at the interface of the PrP dimer in the crystalline structure.

The amplification is generally carried out by incubation of the cells presumed to be infected or not, with an effective amount of compound(s) of general formula I. This incubation is carried out under conditions suitable for the amplification, generally at 37°C, at a physiological pH, for a sufficient period, which may be up to approximately 3 days.

For the purpose of the present invention, the term "effective amount" means at least the minimum and sufficient amount to observe amplification of the oligomerization of at least one PrP^{Sc} isoform possibly present in the biological sample which is the subject of the analysis.

For obvious reasons, this amount can vary significantly depending on the desired sensitivity in terms of the diagnostic method and/or the amount of PrP^{Sc} isoform(s) initially present.

For example, this concentration can range from 0.1 to 20 µM.

The detection may be direct or not.

In particular, the fluorescent properties may be directly used in an ELISA test with direct detection of the interaction between PrP^{c} and a compound according to the invention.

According to a variant, a compound of general formula (I) may furthermore be used in a linked form with:
(i) A luminescent tag like for example Europium, and
(ii)An DNA or RNA fragment that may be amplified by PCR.

This detection may be indirect by using a compound of formula (I) linked to molecules which may be detected by conventional immunologic reactives like the system streptavidin/biotin for example.

The compound of formula (I) may also be used for improving the detection step of conventional dosage, like ELISA dosage, for example.

The detection can be direct or carried out by conventional screening methods such as hybridization or antibody/antigen reaction. Such a treatment requires prior cell lysis using conventional physical, mechanical or chemical methods.

According to a variant of the invention, the cell lysates having undergone the amplification method according to the invention may, prior to the detection of the PrP^{Sc} isoforms, be subjected to a partial digestion with a protease, and in particular protease K, so as to eliminate the PrP^{C} molecules and therefore to conserve only the PrP^{Sc} isoforms in the biological sample. The presence of these PrP^{Sc} isoforms can subsequently be detected by simple conventional analysis, in particular of immunoblotting type.

For example, said PK treatment can be carried out in the following way: 1 µg of PK per 50 or 25 µg of proteins to be digested for 1 h at 37°C for a total of 200 µg of proteins in a final volume of 400 µl.

According to a specific embodiment of the invention, this treatment with protease K can also be carried out under the conditions described in document WO 01/35104, the content of which is incorporated into the present application by way of reference. This treatment aims at completely degrading the normal PrP^{C} isoform while at the same time conserving all or part of the repeated octapeptide motifs of PrP^{Sc}, irrespective of the strain of unconventional transmissible agent (UTA).

It should be noted that a certain number of parameters are tightly linked to one another: the concentration of proteinase K depends directly on the duration of the treatment (incubation time) of the sample: it may thus be considered that, at 37°C for example, a 10 minutes incubation with a PK at a concentration of between 30 and 200 µg/ml of homogenate at 10% is equivalent to a 30 minutes incubation with a PK at a concentration of between 10 µg/ml and 70 µg/ml of homogenate at 10%. For example, a 30 minutes incubation with a PK at 25 µg/ml is equivalent to a 10 minutes incubation with a PK at a concentration of 75 µg/ml.

In the case of the use of a derivative of a compound of general formula I, i.e. provided with a labeling method, the detection can be carried out according to the standard conditions recommended for the marker selected.

A subject of the present invention is also a diagnostic kit useful for implementing a method in accordance with the invention, characterized in that it comprises, as a reagent, at least one compound of general formula I.

According to another of its aspects, the present invention relates to a kit for diagnosing a TSSE, characterized in that it comprises at least one compound of general formula I in combination with a ligand for the pathogenic PrP^{Sc} isoforms. More particularly, it is an antibody which may or may not be monoclonal, or a mixture of monoclonal antibodies.

Such antibodies are in particular described in Nishida et al. (J. Virol, 74:320-325, 2000) and sold by the company Spibio. They are also described in patent application WO 01/35104.

According to another of its aspects, the present invention relates to a kit useful for diagnosing a TSSE, characterized in that it comprises at least one compound of general formula I in the form of a derivative provided with a labeling system. The labeling may be suitable for luminescent detection, in particular by fluorescence, or by affinity, such as a streptavidin/biotin couple, for example.

According to another embodiment, the instant invention is directed to a solid support grafted in surface by at least one compound of general formula I. Such a support may be particularly useful for the depletion of biological medium from pathogen isoform of prion PrP^{Sc}.

The instant invention is also directed to a pharmaceutical composition including as active material at least one compound of general formula I and/or I' as defined previously.

In addition, a subject-matter of the present invention is more particularly, according to another aspect, novel compounds of formula I excepted the 6,6'-(2,2':5',2":5 ",2"'-quaterthiophene-5,5"'-diyl)bis(1,3,5-triazine-2,4-diamine).

These compounds may be easily prepared by the man skilled in the art from commercial products and conventional synthesis reactions in particular by method related to the specific methods disclosed in the following example 1. Halogenous derivatives of thiophene commercially available or obtained by synthesis (for example by cyclization of N-cyanoguanidine with carbonitrile derivatives), react with P spacers according to a double cross coupling reaction (for examples Stille or Suzuki) to afford novel compounds of formula I.

The examples and figures which appear hereinafter are given by way of nonlimiting illustration of the field of the invention:

### Figures:

- Figure 1: It illustrates the activity of a compound I in accordance with the invention, on the PrP^{Sc} from infected N2a58/22L cells.
- Figure 2: It illustrates a comparison between the effect of a compound I in accordance with the invention, on the PrP^{Sc} from infected N2a58/22L cells and that of control compounds identified in example 2, tested using a range of concentration of 20 µM after prolonged incubation of the compounds for 3 days.
- Figure 3: It compares the amplification effect of a compound I according to the invention on the PrP^{Sc} from a prion-infected cellular lysate and that of compound T1 of example 3.

The assays were carried out using, as a compound of general formula I, 6,6'-(2,2':5',2":5",2"'-quaterthiophene-5,5"'-diyl)bis(1,3,5-triazine-2,4-diamine) as compound I.

### Example 1: Preparation of 6,6'-(2,2':5',2":5",2'''-quaterthiophene-5,5'''-diyl)bis(1,3,5-triazine-2,4-diamine) = compound I

All the reactions were carried out in dried or freshly distilled solvents, under a dry argon atmosphere. Commercial reagents have been used without further purification. Pd(PPh₃)₃ was prepared by published methods. Elemental analyses were performed by the Service Central d'Analyse du CNRS (Solaize, France). ¹H NMR spectra were recorded at 300.75 MHz, and ¹³C{¹H} NMR spectra at 75.30 MHz on a Bruker AV-300 instrument. Chemical shifts (δ) were referenced to the residual solvent peak as internal standard. δ and coupling constant values (*J*) are expressed in ppm and Hz, respectively.

### 6-(thiophen-2-yl)-1,3,5-triazine-2,4-diamine (1):

2-thiophenecarbonitrile (1.90 g, 17.4 mmol), N-cyanoguanidine (1.83 g, 21.72 mmol) and KOH (0.10 g, 1.7 mmol) were put in 10 mL of DMSO. The colourless solution was heated at 80°C for 16 h and became yellow. The solvent was removed in vacuum and the crude product was triturated with cooled CH₂Cl₂ (4 x 100 mL at 0 °C) and extract with hot dioxane (100 mL at 100 °C). The solvent was removed in vacuum and **1** was isolated as a white solid. Yield: 2.99 g (89 %).

¹H NMR (DMSO-d₆): δ = 7.80 (dd, *³J_{H-H}* = 3.7, *³J_{H-H}* = 1.0, 1H, Ar), 6.93 (dd, *³J_{H-H}* = 4.8, *³J_{H-H}* = 1.0, 1H, Ar), 6.84 (dd, *³J_{H-H}* = 4.8, *³J_{H-H}* = 3.7, 1H, Ar), 6.76 (br. s, 4H, NH₂ amine).

¹³C{¹H} NMR (DMSO-d₆): δ = 167.1, 166.6 (C₃H₄N₅), 143.0, 130.4, 128.8, 128.0 (C₄H₃S).

### 6-(5-bromothiophen-2-yl)-1,3,5-triazine-2,4-diamine (2):

Under hυ protection, NBS (3.20 g, 12.3 mmol) was added to a solution of 1 (4.87 g, 20.6 mmol) in 50 mL of CH₃COOH at room temperature. After addition the yellow solution was heated to 40 °C for 48 h. The product was precipitated by adding of cooled CH₂Cl₂ (100 mL at 0 °C), filtered over a G4 frit and washed by CH₂Cl₂ (2 x 50 mL, 0 °C) and cyclohexane (2 x 50 mL). The greenish solid was dried in vacuum during 16 h. 2 was isolated has a greenish powder. Yield: 5.05 g (90 %).

¹H NMR (DMSO-d₆): δ = 7.61 (d, *³J_{H-H}* = 4.0, 1H, Ar), 7.28 (d, *³J_{H-H}* = 4.0, 1H, Ar), 6.93 (br. s, 4H, NH₂ amine).

¹³C{¹H} NMR (DMSO-d₆): δ = 166.4, 165.3 (C₃H₄N₅), 144.1, 131.6, 129.4, 116.3 (C₄H₃S).

Anal. Calcd for C₇H₆BrN₅S (272.13): C, 30.90; H, 2.22; Br, 29.36; N, 25.74; S, 11.78. Found: C, 30.40; H, 2.31; Br, 30.79; N, 24.72; S, 11.08.

### 5,5'-bistrimethyltin-2,2'-bithiophene (3):

At -40 °C, 2.25 equivalents of a 1.6 M solution of n-BuLi in n-hexane were added to a solution of bithiophene in THF (C : 0.2 M). The solution was then heated to 25 °C and stirred for 2 h. To the previous white slurry, 3 equivalents of Me₃SnCl) in THF (6 to 10 mL) was added at -40 °C. After 3 h at 25 °C, hexane 50 mL was added and the crude was washed with saturated NH₄Cl (2 x 150 mL) and water (2 x 100 mL). The organic layer was dried over Na₂SO₄, filtered and the solvent was removed in vacuum. The product was checked by ¹H NMR (DMSO-d₆) and used without any other purification. Bis substitutions >98 %; quantitative yield.

¹H NMR (DMSO-d₆): δ = 7.32 (d, *³J_{H-H}* = 3.3, 2H, Ar), 7.13 (d, *³J_{H-H}* = 3.3, 2H, Ar), 0.35 (s, 18H, SnMe₃).

### 6,6'-(2,2':5',2":5",2"'-quaterthiophene-5,5"'-diyl)bis(1,3,5-triazine-2,4-diamine) :

2.05 g of 2 (7.5 mmol) and 3 (1.67 g, 3.4 mmol) were put in a solvent mixture (pyridine 10 mL, DMF 10 mL). Then 5% mol Pd(PPh₃)₃ in 3 mL of THF was added at 70 °C. The yellow solution became orange; the media was heated (110 °C) for 16 h and the colour turn red (blood). At 40 °C, the reaction was quenched by an excess of NBu₄F and the media were stirred for 3 additional hours. At 25°C, hexane (100 mL) was added; a yellow solution and a red precipitate were observed. The crude was filtrated over a G4 frit, washed with hexane (1 x 50 mL), water (2 x 50 mL), MeOH (2 x 50 mL), CH₂Cl₂ (2 x 50 mL) and dried under vacuum. The crude was purified by soxhlet (CH₂Cl₂, MeOH, CH₃CN) and extracted with dioxane afforded the expected compound as a red solid. Yield: 1.10 g (62 %).

¹H NMR (DMSO-d₆): δ = 7.75 (br. s, 2H, Ar), 7.43 (br. s, 2H, Ar), 7.39 (br. s, 4H, Ar), 6.81 (br. s, 4H, NH₂ amine).

¹³C{¹H} NMR (DMSO-d₆): δ = 166.9, 166.0 (C₃H₄N₅), 141.7, 139.3, 135.5, 135.41.7, 139.3, 135.5, 135.4, 129.8, 126.0, 125.7, 124,8 (C₁₆H₈S₄).

Anal. Calcd for C₂₂H₁₆N₁₀S₄ (548.69): C, 48.16; H, 2.94; N, 25.53; S, 23.38. Found: C, 47.66; H, 3.55; N, 22.44; S, 22.56.

### Example 2: In vitro screening on cell cultures chronically infected with prions

The assays are performed *in vitro* on murine neuroblastoma lines chronically infected with the 22L prion strain (scrapie strain stabilized in mice) (Nishida N. et al., J. Virol. (2000) 74, 320-325). This infected cell line, called N2a/22L, produces a large amount of PrP^{Sc} molecules which are resistant to partial digestion with proteinase K and which have properties reminiscent of those of prions. The infected N2a/22L cells are capable of causing a TSSE when they are inoculated intracerebrally into mice. The infected N2a/22L cells are incubated with the compound I, at different concentrations from 1 to 20 µM, for a period of 3 days at 37°C and 5% CO₂.

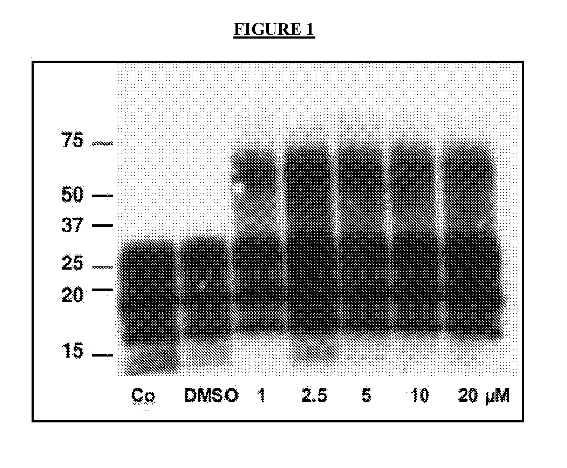

After 3 days, the confluent cells are lyzed in the presence of detergents. The cell lysates, normalized with respect to their amount of proteins, are subjected to partial digestion with proteinase K (PK) in order to eliminate the PrP^{C} molecules. The samples are then analyzed by immunoblotting and the membrane revealed with a mixture af anti-PrP antibodies. The results are given in figure 1.

In figure 1, CO represents the control cells i.e without treatment and DMSO the cells only treated with DMSO. From these results, it appears that the effect of oligomerisation is very important since for a dosis of 2.5µM, the saturation of the signal is achieved.

### Example 3: Comparison of the effect of the compound I and that of other thienyl pyrimidique derivatives

The infected cell lines with the prion as specified in example 2 are incubated with some thienyl pyrimidique derivatives, known for having an oligonerisation activity. Thes compounds which chemical structure is submitted in the following table 1 are tested in the same conditions than the conditions disclosed in example 2 and at a concentration of 20 µM.

| **Product** | **Formulae** | **Oligomeric effect** | **Developed formulae** |
|---|---|---|---|
| **T1** | **C₉H₇BrN₂S₂** | + | 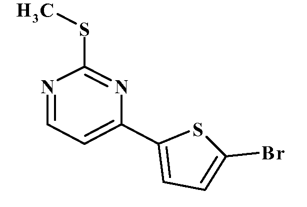 |
| **T2** | **C₁₂H₉N₃S₂** | + | 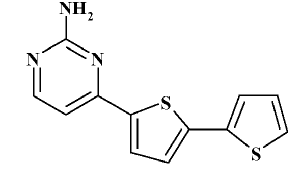 |
| **T3** | **C₁₂H₈N₂S₃** | ++ | 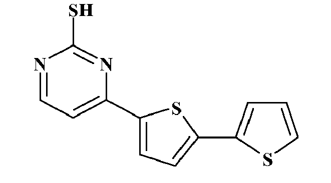 |
| **T4** | **C₉H₈N₂S₂** | - | 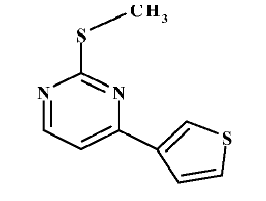 |

The cells infected with prion were incubated (i) with solvent DMSO (ii) with the products T1, T2, T3 and T4 at a concentration of 20 µM for a period of 3 days. (CO corresponds to cells having no treatment).

The confluent cells are lyzed and the cellular extracts are subjected to partial digestion with proteinase K (PK). The samples are then analyzed by immunoblotting with the method disclosed in previous example.

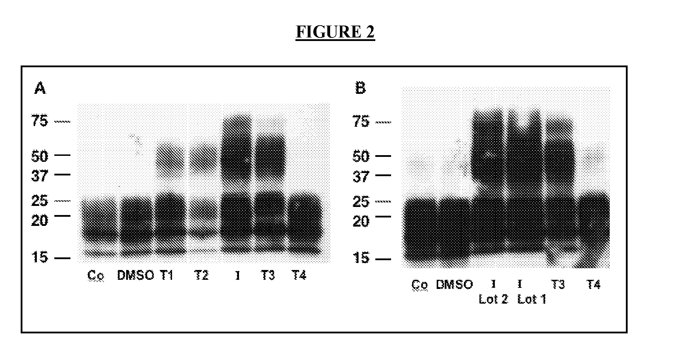

The results, illustrated in figure 2, show that for the compound I, at a concentration of 20 µM, the amount of oligomeric forms observed is much greater than for the compound T 1 or T2 under the same conditions.

### Example 4: Evaluation of the efficiency of the compound I according to the invention on prion-infected cellular lysates

This assay shows that the compound I can oligomerize the pathogen form PrP^{Sc}, directly from cellular extracts incubated for 1 hour at 20°C. For comparative purpose, the same assay is performed with the control compound T1 disclosed in example 2.

The N2a/22L confluent cells are lysed with detergents. The cellular extract is incubated for 1 hour to different concentrations of compounds I and T1 (0,1 to 625 µM) or with only DMSO.

After incubation, the cellular extract is subjected to partial digestion with proteinase K (PK). The samples are then analyzed by immunoblotting with the method disclosed in previous example.

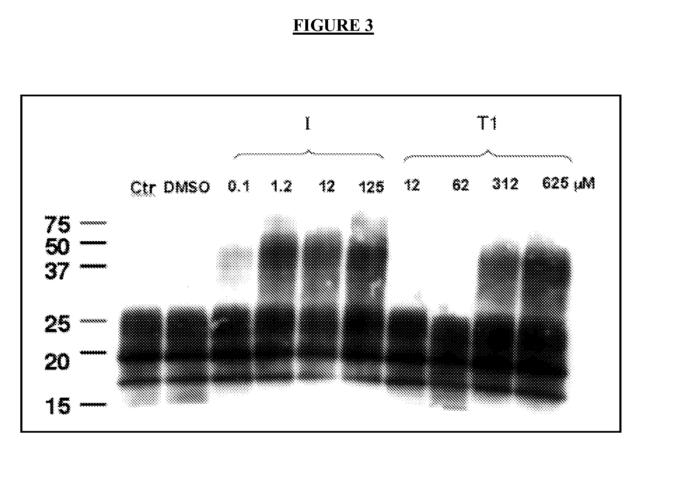

The results are illustrated in figure 3. Ctr represents the untreated cellular extracts.

The analysis by immunoblotting shows that the compound 1 is efficient from 125 µM and that the amplification of the oligomerization in celllular extract is at least 100 times greater than to the concentration of 1µM.

Moreover, the comparison of the oligomeric activity between the compounds I and T1 reveals that for the same amplification signal and greater than 100 times compared to the untreated control extract, the compound I is efficient at 1µM on cellular extract and the compound T1 is only efficient from 312µM.

## Claims

1. A method for selectively amplifying the oligomerization of at least one PrP^{Sc} isoform of the PrP^{C} cellular prion protein, **characterized in that** it comprises bringing said PrP^{Sc} isoform, under conditions suitable for amplification, together with an effective amount of at least one compound of general formula I as follow: 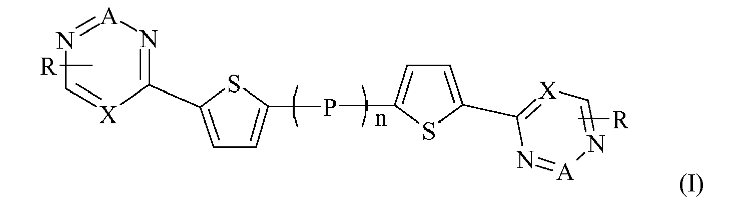 in which:
- A, independently of one another, represent a nitrogen, a -CR₁-, -CO(R₁) -,
- CS(R₁)-, -CN(R₁)(R₂)- group with R₁ and R₂ representing, independently of one another, a hydrogen atom, a saturated or unsaturated, linear, branched or cyclic, C₁ to C₁₂ alkyl radical, where appropriate substituted,
- X, independently of one another, represent a nitrogen atom or CR₁" with R₁" representing a group as defined above for R₁,
- R independently of one another, represent a hydrogen atom or a radical chosen from:
- a OR'₁, N(R'₁)(R'₂), SR'₁ group with R'₁ and R'₂ representing, independently of one another, a group as defined above for R₁ and/or R₂,
- a saturated or unsaturated, linear, branched or cyclic, C₁ to C₁₂ hydrocarbon-based radical, the hydrocarbon-based chain of which may, where appropriate, be interrupted with one or more heteroatoms chosen from S, O and N atoms, and where appropriate, substituted for example by a OR'₁, N(R'₁)(R'₂), SR'₁ group as defined previously
- P represents an aromatic nucleus C₅ to C₁₆ or a saturated or unsaturated linear, branched or cyclic C₁ to C₁₂ alkyl divalent group, the hydrocarbon-based chain of which may, where appropriate, be interrupted with one or more heteroatoms chosen from S, O, N and Si atoms, and/or with one or more cyclic alkyl ring, aryl ring or heteroaryl ring and where appropriate, substituted,
- n is zero or an integer equal or greater to 1,
and salts, solvates, isomers or derivatives thereof.

2. The method of claim 1 wherein P is a 5- or 6-membered aromatic ring comprising 1 or 2 heteroatoms or a bi- or tri-cyclic aromatic ring comprising from 1 to 4 heteroatoms.

3. The method according to claim 1 or 2 wherein P is chosen from pyrrolyl, imidazolyl, pyridyl, pyrimidinyl, carbazolyl, pyrazinyl, indolyl, indazolyl, furyl, pyranyl, thiazolyl, thiopuranyl and thienyl ring.

4. The method as claimed in any one of the preceding claims, wherein the radicals R are in the meta- or para-position with respect to the groups A.

5. The method as claimed in any one of the preceding claims, wherein A represents a -CN(R₁)(R₂)- divalent group with R₁ and R₂ representing an alkyl radical as defined in claim 1.

6. The method as claimed in any one of the preceding claims, wherein the compound is of formula (I'): 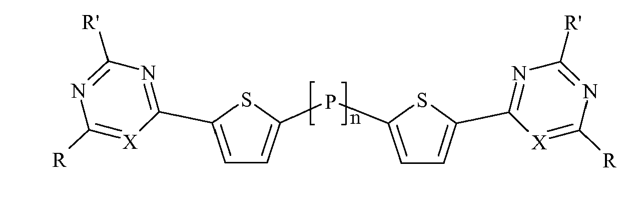 with R, P, n and X being as defined in anyone of the previous claims and R' being a radical chosen among NR'₁R'₂ and SR'₁ with R'₁ and R'₂ being as defined in claim 1.

7. The method as claimed in any one of the preceding claims, wherein R represent an N(R'₁)(R'₂).

8. The method as claimed in any one of the preceding claims, wherein X represent a nitrogen.

9. The method as claimed in any one of the preceding claims, wherein n is different from zero.

10. The method according to claim 1 to 9 wherein P is a thienyl ring, where appropriate substituted.

11. The method as claimed in any one of the preceding claims, wherein n is equal to at least 2 when P is a thienyl ring.

12. The method as claimed in any one of the preceding claims, wherein the compound of formula I is 6,6'-(2,2':5',2":5",2"'-quaterthiophene-5,5"'-diyl)bis(1,3,5-triazine-2,4-diamine) 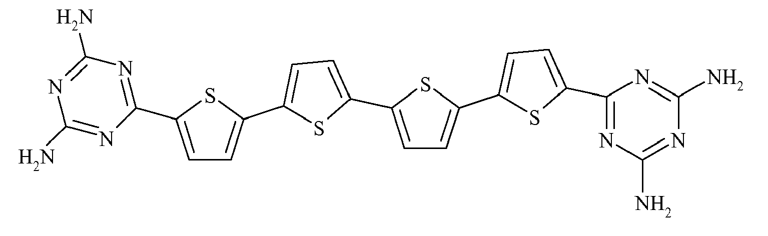 or a salt or derivative thereof.

13. The method as claimed in any one of the preceding claims, wherein said compound of general formula I is in the form of a derivative thereof incorporating at least one entity capable of providing it with a method of detection and/or promoting the linking of said compound to a solid support.

14. The method as claimed in the preceding claim, wherein said entity is a marker suitable for luminescent detection or detection by affinity.

15. The method as claimed in any one of the preceding claims, wherein the amplification is carried out in the presence of the PrP^{C} isoform.

16. A method useful for detecting a PrP^{Sc} isoform of the PrP^{C} cellular prion protein, comprising at least the implementation of a method as defined in any one of claims 1 to 14 and of a step of detection of said isoform.

17. A method useful for detecting the presence or the risk of the occurrence of an encephalopathy in an individual, said method comprising at least:
- bringing a biological sample from said individual together with an effective amount of at least one compound of general formula I as defined in claims 1 to 14, under conditions suitable for the amplification of PrP^{Sc} isoform(s) possibly present, and
- detecting the possible presence of said PrP^{Sc} isoform, indicative of the presence or of the risk of development of an encephalopathy in said individual.

18. The method as claimed in previous claim, useful for detecting a transmissible subacute spongiform encephalopathy (TSSE).

19. The method as claimed in claim 17 or 18, useful for detecting a Creutzfeldt-Jakob disease in humans, scrapie in sheep and goat, and bovine spongiform encephalopathy.

20. The method as claimed in any one of claims 18 to 19, wherein the compound of general formula I is used in the form of a derivative provided with a labeling system.

21. The method as claimed in any one of claims 14 to 20, wherein in addition, the treatment of all or part of said sample treated with a protease so as to destroy the normal PrP^{C} isoform, the separation of proteins, and the detection of PrP^{Sc} isoform, possibly present, using a specific antibody, are carried out.

22. The method as claimed in the preceding claim, wherein the protease used is protease K.

23. The method as claimed in the preceding claim, wherein the treatment with protease K is carried out under conditions suitable for complete degradation of the PrP^{C} protein while at the same time conserving all or part of the PrP^{Sc} protein, and in that the subsequent detection of PrP^{Sc} isoform(s) is carried out.

24. A kit useful for selectively amplifying the oligomerization of at least one PrP^{Sc} isoform of the PrP^{C} cellular prion protein, **characterized in that** it comprises, as a reagent, at least one compound of general formula I as defined in claims 1 to 14.

25. A kit useful for diagnosing encephalopathies, in particular animal or human transmissible subacute spongiform encephalopathies (TSSEs), comprising, as a reagent, at least one compound of general formula I as defined in claims 1 to 14 in the form of a derivative provided with a labeling system.

26. A kit useful for diagnosing encephalopathies, and in particular animal or human transmissible subacute spongiform encephalopathies (TSSEs), comprising, as a reagent, at least one compound of general formula I as defined in claims 1 to 14 in combination with a ligand which binds specifically to a PrP isoform.

27. A kit as claimed in claim 25 or 26, **characterized in that** it also comprises an antibody or a mixture of antibodies recognizing a PrP^{Sc} isoform.

28. Solid support grafted in surface by at least one compound as defined in anyone of claims 1 to 14.

29. Use of a support as defined in previous claim for the depletion of biological medium from pathogen isoform of prion PrP^{Sc}.

30. A pharmaceutical composition including as active material at least one compound of general formula I as follow: 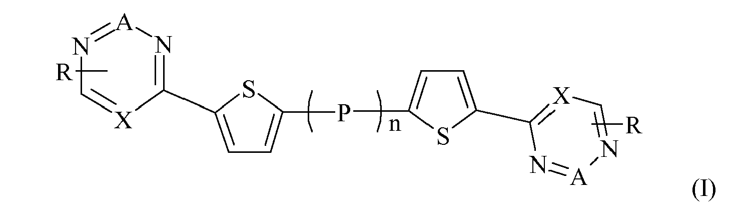 in which:
- A, independently of one another, represent a nitrogen, a -CR₁-, -CO(R₁) -,
- CS(R₁)-, -CN(R₁)(R₂)- group with R₁ and R₂ representing, independently of one another, a hydrogen atom, a saturated or unsaturated, linear, branched or cyclic, C₁ to C₁₂ alkyl radical, where appropriate substituted,
- X, independently of one another, represent a nitrogen atom or CR₁" with R₁" representing a group as defined above for R₁,
- R, independently of one another, represent a hydrogen atom or a radical chosen from:
- a OR'₁, N(R'₁)(R'₂), SR'₁ group with R'₁ and R'₂ representing, independently of one another, a group as defined above for R₁ and/or R₂,
- a saturated or unsaturated, linear, branched or cyclic, C₁ to C₁₂ hydrocarbon-based radical, the hydrocarbon-based chain of which may, where appropriate, be interrupted with one or more heteroatoms chosen from S, O and N atoms, and where appropriate, substituted for example by a OR'₁, N(R'₁)(R'₂), SR'₁ group as defined previously ,
- P represents an aromatic nucleus C₅ to C₁₆ or a saturated or unsaturated linear, branched or cyclic C₁ to C₁₂ alkyl divalent group, the hydrocarbon-based chain of which may, where appropriate, be interrupted with one or more heteroatoms chosen from S, O, N and Si atoms, and/or with one or more cyclic alkyl ring, aryl or heteroaryl ring and where appropriate, substituted,
- n is zero or an integer equal or greater to 1,
and salts, solvates, isomers or derivatives thereof.

31. The pharmaceutical composition according to the previous claim wherein said compound is as defined in anyone of the claims 2 to 14.

32. The pharmaceutical composition according to claim 30 or 31 wherein the compound is the 6,6'-(2,2':5',2":5",2'''-quaterthiophene-5,5'''-diyl)bis(1,3,5-triazine-2,4-diamine or salts, solvates, isomers or derivatives thereof.

33. A compound of general formula I as follow: 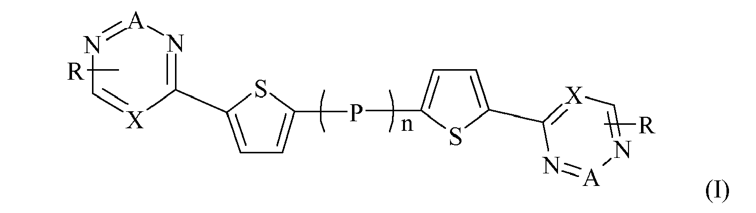 in which:
- A, independently of one another, represent a nitrogen, a -CR₁-, -CO(R₁) -,
- CS(R₁)-, -CN(R₁)(R₂)- group with R₁ and R₂ representing, independently of one another, a hydrogen atom, a saturated or unsaturated, linear, branched or cyclic, C₁ to C₁₂ alkyl radical, where appropriate substituted,
- X, independently of one another, represent a nitrogen atom or CR₁" with R₁" representing a group as defined above for R₁,
- R, independently of one another, represent a hydrogen atom or a radical chosen from:
- a OR'₁, N(R'₁)(R'₂), SR'₁ group with R'₁ and R'₂ representing, independently of one another, a group as defined above for R₁ and/or R₂,
- a saturated or unsaturated, linear, branched or cyclic, C₁ to C₁₂ hydrocarbon-based radical, the hydrocarbon-based chain of which may, where appropriate, be interrupted with one or more heteroatoms chosen from S, O and N atoms, and where appropriate, substituted for example by a OR'₁, N(R'₁)(R'₂), SR'₁ group as defined previously ,
- P represents an aromatic nucleus C₅ to C₁₆ or a saturated or unsaturated linear, branched or cyclic C₁ to C₁₂ alkyl divalent group, the hydrocarbon-based chain of which may, where appropriate, be interrupted with one or more heteroatoms chosen from S, O, N and Si atoms, and/or with one or more cyclic alkyl ring, aryl or heteroaryl ring and where appropriate, substituted,
- n is zero or an integer equal or greater to 1,
and salts, solvates, isomers or derivatives thereof except the 6,6'-(2,2':5',2":5",2"'-quaterthiophene-5,5'''-diyl)bis(1,3,5-triazine-2,4-diamine.

34. A compound according to the previous claim wherein X, A, R, P and n are as defined in anyone of the claims 2 to 14.
